# FASCICULE DE BREVET EUROPEEN

(11) **EP 3 603 574 B1**
(45) Date de publication et mention de la délivrance du brevet: **17.04.2024**
(21) Numéro de dépôt: 18187345.6
(22) Date de dépôt: 03.08.2018
(51) Int. Cl.: A61F 2/08

(54) **DISPOSITIF D'ANCRAGE OSSEUX**
KNOCHENVERANKERUNGSVORRICHTUNG
BONE ANCHORING DEVICE

(43) Date de publication de la demande: 05.02.2020
(73) Titulaire: Keri Medical SA, 1227 Les Acacias (CH)
(72) Inventeur: PRANDI, Bernard, 6006 Luzern (CH); MOTTET, Julie, 74930 Pers-Jussy (FR); MOTTE, Damien, 74000 Annecy (FR); MRAOVIC, Tanguy, 66540 Baho (FR)
(74) Mandataire: Micheli & Cie SA

(56) Documents cités:
- WO-A1-94/28799
- WO-A1-95/18571
- US-A- 5 891 168
- US-A1- 2010 292 715
- US-A1- 2015 165 096

## Description

La présente invention concerne un dispositif d'ancrage destiné à être fixé dans un os lors d'une intervention relevant de la chirurgie orthopédique et notamment un dispositif tel qu'une ancre de suture ou un implant d'arthrodèse.

Les lésions ligamentaires et tendineuses sont parmi les affections les plus fréquemment rencontrées en orthopédie et peuvent toucher un grand nombre d'articulations différentes. L'un des traitements connu de ces lésions consiste à venir refixer les tendons ou ligaments endommagés à leur place sur l'os grâce à une ancre et à un fil de suture. Pour ce faire, un perçage est réalisé dans l'os, l'ancre est ancrée dans l'os et un fil qui lui est accroché permet ensuite de suturer le tissu endommagé. Il existe des ancres chassées, vissées ou « à noeud » qui peuvent-être résorbables ou non résorbables. L'invention concerne en particulier une ancre de suture de type chassée non résorbable.

Lors d'une telle chirurgie, il est préférable de préserver au maximum l'os du patient et donc de disposer d'une ancre apte à s'insérer dans un perçage diamétralement petit et peu profond, en particulier lorsqu'elle concerne un petit os tel qu'une phalange. Une telle ancre doit également permettre un ancrage performant, résistant aux forces de traction et aux forces cycliques exercées par les tendons ou les ligaments.

En outre, seul le chirurgien sait apprécier, au moment de l'intervention, la taille idéale du fil nécessaire pour suturer les tissus, il est donc avantageux que le fil de suture attaché à l'ancre soit accessible et facilement interchangeable. Or, dans de nombreuses ancres de l'art antérieur, le fil de suture passe à travers une ouverture à contour fermé de très petite dimension si bien que le fil de suture est soit monté en usine, l'ancre étant livrée avec un fil pré-monté au chirurgien, soit inséré par le chirurgien à l'aide d'un passe fil.

Enfin, certains ligaments ont plusieurs terminaisons et nécessitent donc plusieurs points d'ancrage. Pour éviter d'insérer plusieurs ancres de suture, une ancre multi-fils peut parfois s'avérer nécessaire.

La demande internationale WO9942064 A1 décrit une ancre de suture non résorbable destinée à être chassée dans l'os. Pour cette ancre, l'ancrage dans la masse osseuse est réalisé au moyen d'ailettes s'étendant radialement par rapport à l'axe longitudinal de l'ancre. Le déploiement de l'ancre décrite étant relativement faible, celle-ci ne semble pas assurer un ancrage résistant. De plus, il semble qu'elle risque d'endommager l'os cortical.

L'arthrose, qu'elle soit primaire, lorsque les patients ne présentent pas de prédisposition évidente, ou secondaire, lorsqu'elle est la conséquence directe de maladies articulaires, est également une affection courante en orthopédie. Elle touche particulièrement les articulations fortement sollicitées, notamment celles de la main telle que l'articulation inter-phalangienne distale (ou IPD).

A ce jour, aucune solution d'arthroplastie IPD ne s'est montrée véritablement efficace. Ainsi, l'arthrodèse est une opération courante sur cette articulation car elle n'handicape pas complètement le patient et permet l'indolence. Elle a pour but de bloquer la mobilité articulaire du patient et de construire un pont par fusion osseuse entre la phalange distale P3 et la phalange médiane P2, pour éliminer l'arthrose articulaire.

Il existe différents types d'implants pour l'arthrodèse de l'IPD: l'implant intramédullaire monobloc ou multi-pièce qui s'insère par voie dorsale, les vis de compression qui s'insèrent par voie pulpaire, et les broches orthopédiques de Kirschner (k-wires). Les risques d'infections par voie pulpaire étant plus importants que par voie dorsale, l'implant intramédullaire, de préférence monobloc est préférable. En outre, traverser la pulpe peut endommager la sensibilité du doigt, ce que l'on préfère bien sûr éviter.

Ce type d'implant comprend deux zones d'ancrage osseux de part et d'autre d'une zone rigide. Ces deux zones d'ancrage sont destinées à être introduites dans des trous calibrés réalisés dans les os à joindre et à s'y ancrer de manière suffisamment forte pour assurer une bonne accroche dans l'os.

Comme pour les ancres de suture, les contraintes s'appliquant aux parties d'ancrage des implants d'arthrodèse, par exemple pour arthrodèse IPD, doivent répondre à de nombreux critères incluant une petite taille lors de l'insertion (perçage diamétralement petit et peu profond) et un ancrage fort pour résister aux contraintes associées à l'articulation. La réalisation d'un perçage de petite taille est particulièrement importante dans le cas d'une arthrodèse car une bonne préservation de l'os facilite la fusion osseuse.

La demande de brevet française FR 2 913 876 A1 concerne un dispositif pour arthrodèse intra-médullaire comprenant deux zones d'ancrage. Chacune de ces zones d'ancrage comprend deux bras définissant entre eux une fente s'étendant selon un axe et agencés de sorte que l'envergure de la zone d'ancrage perpendiculairement à cet axe puisse, à partir de ladite position de repos, être réduite en rapprochant élastiquement lesdits deux bras l'un de l'autre, ladite zone d'ancrage étant destinée à être contrainte en position repliée pour son insertion dans un logement réalisé dans l'os, typiquement un trou centromédullaire calibré, puis à être relâchée pour s'ancrer dans l'os sous l'effet de forces de rappel élastiques. Cependant, la structure de cet implant impose, même dans sa position fermée, un certain diamètre pour le logement dans l'os qu'il serait intéressant de diminuer. De plus, la forme de cet implant est telle que seules les extrémités des bras vont venir en appui contre la corticale et risquent de l'endommager.

Des dispositifs d'ancrage ayant une fente et deux bras sont connus des documents US-A-2015/165096 et US-A-2010/292715.

Dans un dispositif d'ancrage osseux tel qu'une ancre de suture ou un implant d'arthrodèse, destiné à être utilisé en orthopédie, la partie à ancrer dans l'os doit pouvoir prendre un minimum de place lors de son insertion pour permettre la réalisation d'un perçage ou d'un trou d'envergure minimum tout en permettant un ancrage fort, apte à résister à de fortes contraintes mécaniques. Un premier objectif de l'invention est de proposer un dispositif présentant ces propriétés.

Dans le cas particulier des ancres de suture, un second objectif est également de proposer un dispositif d'ancrage permettant une insertion facile du fil de suture et la possibilité de la relier à plusieurs fils de suture si besoin.

L'invention propose à ces fins un dispositif d'ancrage osseux comprenant un premier et un second bras définissant entre eux une première fente de profondeur p s'étendant selon un axe x-x', lesdits premier et second bras portant respectivement une première et une seconde branche s'étendant à l'extérieur de ladite première fente, ladite première branche étant agencée de manière à définir avec le bras qui la porte une seconde fente et ladite seconde branche étant agencée de manière à définir avec le bras qui la porte une troisième fente, ledit dispositif étant agencé de sorte que, en position de repos :
- la projection orthogonale sur l'axe (x-x') du fond de chacune des seconde (3b ; 13b) et troisième (3c ; 13c) fentes se situe dans ladite première fente (3a; 13a) à une distance du fond de cette fente d'au moins 10%, de préférence d'au moins 20%, de préférence d'au moins 30%, de préférence d'au moins 40%, de préférence encore d'au moins 50% de la profondeur p ; et
- les distances d1 séparant le fond de ladite seconde fente de la partie de l'extrémité de la branche qui la définie qui en est la plus éloignée et d2 séparant le fond de ladite troisième fente de la partie de l'extrémité de la branche qui la définie qui en est la plus éloignée soient telles que chacun des ratios d1/p et d2/p soit supérieur ou égal à 0,3, de préférence supérieur ou égal à 0,4, de préférence encore supérieur ou égal à 0,5 ;
ledit dispositif étant apte à diminuer l'envergure de l'ensemble comprenant lesdits premier et second bras et lesdits première et seconde branches perpendiculairement à l'axe x-x' à partir de ladite position de repos, d'une part par le rapprochement élastique de chacune des branches du bras qui la porte et d'autre part par le rapprochement élastique desdits premier et second bras l'un de l'autre, pour son insertion, au moins en partie, dans un os.

Lorsque l'envergure de l'ensemble comprenant lesdits premier et second bras et lesdits première et seconde branches perpendiculairement à l'axe x-x' est diminuée, on dit que cet ensemble est en position repliée, lorsqu'elle est diminuée au maximum, on dit que cet ensemble est en position repliée extrême.

L'ensemble comprenant les bras et branches du dispositif d'ancrage selon l'invention est destiné à être maintenu en position repliée voire en position repliée extrême par le chirurgien, typiquement à l'aide d'une pince ou d'un autre instrument, pour son insertion dans un trou préalablement réalisé dans un os. Une fois ledit ensemble replié en place dans l'os, lorsque le chirurgien libère le dispositif de son instrument, il se déploie doublement c'est-à-dire à la fois par écartement de ses bras l'un de l'autre et par écartement des branches par rapport aux bras, perpendiculairement à l'axe x-x', pour permettre un ancrage fort du dispositif dans l'os.

L'envergure de l'ensemble comprenant lesdits premier et second bras et lesdites première et seconde branches perpendiculairement à l'axe x-x' peut typiquement, lors desdits rapprochements élastiques, être réduite d'au moins 30% par rapport à ladite envergure en position de repos, c'est-à-dire multipliée par un coefficient de réduction « k » inférieur ou égal à 0,7. Elle peut de préférence être multipliée par un coefficient de réduction k inférieur ou égal à 0,6, de préférence encore inférieur à 0,55.

Un faible coefficient de réduction k de cette envergure présente l'avantage de permettre l'insertion de l'ensemble comprenant les bras et branches du dispositif d'ancrage selon l'invention dans un trou d'envergure minimum tout en conférant audit dispositif un ancrage fort, apte à résister à de fortes contraintes mécaniques.

Avantageusement, le dispositif d'ancrage selon l'invention est conçu de sorte que l'ensemble comprenant ses bras et branches soit apte à se déployer d'une première position repliée dans laquelle son envergure perpendiculairement à l'axe x-x' est inférieure ou égale à 0,7, de préférence inférieure ou égale à 0,6, de préférence encore inférieure ou égale à 0,55 fois son envergure perpendiculairement à l'axe x-x' en position de repos, jusqu'à une seconde position dans laquelle son envergure perpendiculairement à l'axe x-x' est supérieure ou égale à 0,75, de préférence 0,80, de préférence 0,85, de préférence 0,90, de préférence 0,95 fois son envergure perpendiculairement à l'axe x-x' en position de repos, de préférence encore jusqu'à sa position de repos, dans les matériaux dont la pression à la rupture en compression est inférieure ou égale à 10 MPa, de préférence inférieure ou égale à 15 MPa, tout en étant incapable de se déployer même partiellement dans les matériaux dont la pression à la rupture en compression est supérieure ou égale à 200 MPa, de préférence supérieure ou égale à 100 MPa, de préférence supérieure ou égale à 50 MPa.

Ces conditions permettent de garantir le déploiement de l'ensemble comprenant les bras et branches du dispositif d'ancrage selon l'invention lorsqu'il se trouve dans l'os spongieux sans risquer d'endommager la partie corticale de l'os.

Avantageusement, dans ladite position de repos, au moins 60%, de préférence au moins 70%, de préférence au moins 80%, de préférence au moins 90%, de préférence encore au moins 95% de la projection orthogonale sur l'axe x-x' de chacune desdites première et seconde branches se situe dans ladite première fente. De manière encore plus préférée, dans ladite position de repos la projection orthogonale sur l'axe x-x' de chacune desdites première et seconde branches se situe complètement ou presque complètement dans ladite première fente. Cette caractéristique participe à l'obtention d'un coefficient de réduction d'envergure k faible car elle permet une addition maximale des réductions d'envergure dues d'une part au rapprochement élastique de chacune des branches du bras qui la porte et d'autre part du rapprochement élastique desdits premier et second bras l'un de l'autre.

Avantageusement, le dispositif d'ancrage osseux selon l'invention est monobloc.

Le dispositif d'ancrage osseux selon l'invention est typiquement réalisé en un métal biocompatible tel que le titane ou en un alliage nickel/titane. Il peut également être réalisé par exemple en un polymère tel que le PE ou le PEEK ou en fibres polyester. Il est de préférence réalisé en un alliage à mémoire de forme nickel/titane tel que le nitinol, typiquement 55%nickel/45%titane. Un tel matériau présente l'avantage d'être superélastique.

De préférence, les surfaces avant et arrière desdits premier et second bras et desdites première et seconde branches sont planes et parallèles à un même premier plan incluant l'axe x-x'. De préférence encore, les surfaces avant et arrière de l'ensemble du dispositif d'ancrage osseux selon l'invention sont planes et parallèles audit premier plan. De cette façon, il est possible de fabriquer un dispositif d'ancrage osseux selon l'invention au moyen d'un procédé de fabrication simple, par découpage, typiquement au laser, à partir de plaques de matériau, par exemple à partir de plaques de nitinol.

Avantageusement, l'ensemble comprenant lesdits premier et second bras et lesdites première et seconde branches présente un plan de symétrie orthogonale, ce plan incluant typiquement l'axe x-x'. Un tel plan de symétrie augmente la stabilité du dispositif d'ancrage une fois dans l'os et permet donc un meilleur ancrage. De préférence, l'ensemble du dispositif d'ancrage osseux selon l'invention est symétrique par rapport audit plan de symétrie orthogonale.

Une méthode pour la mise en place d'un dispositif d'ancrage osseux selon l'invention comprend au moins les étapes suivantes:
A. réaliser un trou s'étendant au moins dans la partie spongieuse d'un os ;
B. diminuer l'envergure de l'ensemble comprenant lesdits premier et second bras et lesdites première et seconde branches perpendiculairement à l'axe x-x' d'au moins 30%, de préférence d'au moins 40%, de préférence encore d'au moins 45% par rapport à ladite envergure en position de repos, d'une part par le rapprochement élastique de chacune des branches du bras qui la porte et d'autre part par le rapprochement élastique desdits premier et second bras l'un de l'autre ;
C. insérer l'ensemble comprenant lesdits premier et second bras et lesdites première et seconde branches dans ledit trou en maintenant son envergure telle que définie dans l'étape B ; et
D. arrêter le maintien de l'envergure telle que définie dans l'étape B de sorte que ledit ensemble se déploie au moins partiellement dans la partie spongieuse dudit os, l'envergure dudit ensemble perpendiculairement à l'axe x-x' augmentant typiquement sous l'effet de forces de rappel élastique au moins jusqu'à atteindre 75%, de préférence 80%, de préférence 85%, de préférence 90%, de préférence encore 95% voire 100% de son envergure en position de repos.

Dans un premier mode de réalisation de l'invention, le dispositif d'ancrage osseux est une ancre de suture orthopédique, ladite première fente étant destinée à recevoir un ou plusieurs fils de suture pour suturer un tissu tel qu'un ligament ou un tendon.

Une ancre de suture selon le premier mode de réalisation de l'invention présente l'avantage de pouvoir s'insérer dans un os dans un trou de petite envergure et de pouvoir s'ancrer solidement dans cet os grâce à la double expansion de ses bras et de ses branches une fois dans l'os, pour suturer un tissu endommagé. Une telle ancre de suture présente également l'avantage de permettre une insertion du ou des fils de suture par le chirurgien, en fonction de son besoin, sans nécessiter de passe-fil.

De préférence, cet os est choisi parmi de petits os tels que ceux de la main, du poignet ou du pied. De préférence encore, il s'agit d'une phalange, par exemple d'une phalange distale P3.

Une méthode pour la mise en place d'une ancre de suture orthopédique selon l'invention comprend typiquement les étapes suivantes:
- réaliser un trou traversant de part en part la partie corticale d'un os et s'étendant dans la partie spongieuse dudit os ;
- positionner un ou plusieurs fils de suture dans la première fente de ladite ancre de suture ;
- diminuer l'envergure de l'ensemble comprenant lesdits premier et second bras et lesdites première et seconde branches perpendiculairement à l'axe x-x' d'au moins 30%, de préférence d'au moins 40%, de préférence encore d'au moins 45% par rapport à ladite envergure en position de repos, d'une part par le rapprochement élastique de chacune des branches du bras qui la porte et d'autre part par le rapprochement élastique desdits premier et second bras l'un de l'autre ;
- insérer l'ensemble comprenant lesdits premier et second bras et lesdites première et seconde branches dans ledit trou en maintenant son envergure telle que définie dans l'étape précédemment décrite ;
- arrêter le maintien de ladite envergure tel que dans l'étape précédemment décrite de sorte que ledit ensemble se déploie au moins partiellement dans la partie spongieuse dudit os, l'envergure dudit ensemble perpendiculairement à l'axe x-x' augmentant typiquement sous l'effet de forces de rappel élastique au moins jusqu'à atteindre 75%, de préférence 80%, de préférence 85%, de préférence 90%, de préférence encore 95% voire 100% de son envergure en position de repos ; et
- suturer un tissu tel qu'un ligament ou un tendon avec le ou les fils de suture.

Dans un second mode de réalisation de l'invention, le dispositif d'ancrage osseux selon le second mode de réalisation de l'invention comprend une première partie d'ancrage comprenant lesdits premier et second bras et lesdites première et seconde branches et destinée à être ancrée dans un premier os, et une seconde partie d'ancrage, rendue solidaire de la première par l'intermédiaire d'une portion rigide centrale et destinée à être ancrée dans un second os, ladite seconde partie d'ancrage pouvant être de forme identique ou différente de ladite première partie d'ancrage. Il s'agit typiquement d'un implant pour arthrodèse, de préférence un implant intramédullaire pour arthrodèse inter-phalangienne distale.

Un tel dispositif d'ancrage osseux permet la construction d'un pont osseux solide entre lesdits premier et second os. Au moins ladite première partie d'ancrage présente l'avantage de pouvoir s'insérer dans ledit premier os dans un trou de petite envergure et de pouvoir s'ancrer solidement dans cet os grâce à la double expansion de ses bras et de ses branches.

De préférence, ces deux os sont choisis parmi de petits os tels que ceux de la main, du poignet ou du pied. De préférence encore, ledit premier os est une phalange distale P3 et ledit second os correspond à la phalange médiane P2 associée biologiquement à ladite phalange P3.

Une méthode pour la mise en place d'un implant pour arthrodèse selon l'invention comprend typiquement les étapes suivantes:
- réaliser un premier trou dans la partie spongieuse d'un premier os d'une articulation entre deux os à traiter et un second trou dans la partie spongieuse du second os de ladite articulation ;
- ancrer la seconde partie d'ancrage dans le second trou ; et
- ancrer la première partie d'ancrage dans le premier trou en réalisant les étapes suivantes :
   ∘ diminuer l'envergure de l'ensemble comprenant lesdits premier et second bras et lesdites première et seconde branches perpendiculairement à l'axe x-x' d'au moins 30%, de préférence d'au moins 40%, de préférence encore d'au moins 45% par rapport à ladite envergure en position de repos, d'une part par le rapprochement élastique de chacune des branches du bras qui la porte et d'autre part par le rapprochement élastique desdits premier et second bras l'un de l'autre ;
   ∘ insérer l'ensemble comprenant lesdits premier et second bras et lesdites première et seconde branches dans ce trou en maintenant son envergure telle que définie dans l'étape précédemment décrite ; et
   ∘ arrêter le maintien de l'envergure tel que dans l'étape précédemment décrite de sorte que ledit ensemble se déploie au moins partiellement dans la partie spongieuse dudit premier os, l'envergure dudit ensemble perpendiculairement à l'axe x-x' augmentant typiquement sous l'effet de forces de rappel élastique au moins jusqu'à atteindre 75%, de préférence 80%, de préférence 85%, de préférence 90%, de préférence encore 95% voire 100% de son envergure en position de repos.

L'invention concerne également un kit comprenant un dispositif d'ancrage osseux selon l'invention et un instrument apte à porter ledit dispositif et à le maintenir au moins dans une position dans laquelle l'envergure perpendiculairement à l'axe x-x' de l'ensemble comprenant lesdits premier et second bras et lesdites première et seconde branches est réduite d'au moins 30% par rapport à ladite envergure en position de repos.

D'autres caractéristiques et avantages de la présente invention apparaîtront à la lecture de la description détaillée suivante faite en référence aux dessins annexés dans lesquels :
- les figures 1a et 1b représentent en perspective un dispositif d'ancrage selon un premier mode de réalisation de l'invention respectivement dans une première position dite « position déployée » et dans une seconde position dite « position repliée extrême » ;
- les figures 2a et 2b représentent, en vue de face, le dispositif d'ancrage représenté aux figures 1a et 1b respectivement dans ladite position déployée et dans ladite position repliée extrême ;
- les figures 3a et 3b représentent, en vue de dessus, le dispositif représenté aux figures 1a et 1b respectivement dans ladite position déployée et dans ladite position repliée extrême ;
- les figures 4a et 4b représentent, en perspective, le dispositif représenté aux figures 1a et 1b en position déployée respectivement avec un ou deux fils de suture en place ;
- les figures 5a et 5b représentent des étapes successives lors de l'insertion du dispositif représenté aux figures 1a et 1b à travers un trou réalisé dans un os ; la figure 5c illustre les contours d'un trou tel que représenté aux figures 5a et 5b dans une coupe transversale d'un os tel qu'une phalange P3 d'un doigt de la main ;
- la figure 6a représente en perspective, un instrument conçu pour la préhension et l'insertion du dispositif représenté aux figures 1a et 1b dans le trou représenté aux figures 5a à 5c ; les figures 6b et 6c représentent, par transparence, le dispositif représenté aux figures 1a et 1b dans des positions respectivement déployée et repliée extrême, à l'intérieur de l'instrument représenté à la figure 6a. Pour des raisons de lisibilité, seule une partie dite active de l'instrument est représentée dans ces figures 6b et 6c ;
- les figures 7a et 7b représentent en vue de face un dispositif d'ancrage selon un second mode de réalisation de l'invention respectivement dans une première position dite « position déployée » et dans une seconde position dite « position repliée extrême » ;
- les figures 7c et 7d représentent, en vue de dessus, le dispositif représenté aux figures 7a et 7b respectivement dans ladite position déployée et dans ladite position repliée extrême ;
- les figures 8a et 8b représentent respectivement en perspective et de côté le dispositif d'ancrage représenté aux figures 7a et 7b dans ladite position déployée ;
- les figures 8c et 8d représentent respectivement en perspective et en vue de côté, une première variante du dispositif représenté aux figures 7a et 7b ;
- les figures 8e et 8f représentent respectivement en perspective et en vue de côté, une seconde variante du dispositif représenté aux figures 7a et 7b.
- les figures 9a et 9b représentent des étapes successives lors de l'insertion du dispositif représenté aux figures 7a et 7b à travers un trou calibré réalisé dans l'os ; la figure 9c illustre les contours d'un trou tel que représenté aux figures 9a et 9b dans une coupe transversale d'un os tel qu'une phalange P3 d'un doigt de la main ;
- la figure 10 représente en perspective et par transparence, le dispositif représenté aux figures 7a et 7b en position dans l'os.

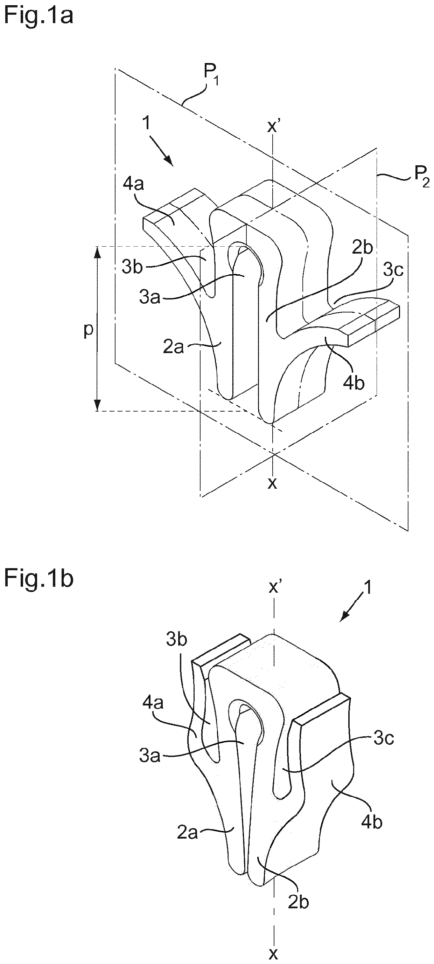

En référence aux figures 1a à 5b, un dispositif d'ancrage osseux selon un premier mode de réalisation de l'invention est une ancre de suture 1 destinée à être utilisée pour refixer un tissu tel qu'un tendon ou un ligament sur un os, typiquement sur une phalange distale (P3) d'un doigt de la main.

L'ancre de suture 1 comprend un premier 2a et un second 2b bras, typiquement agencés en forme de U ou de V, définissant entre eux une première fente 3a. Cette première fente 3a a une profondeur de longueur p et s'étend selon un axe x-x', x correspondant à la partie inférieure et x' à la partie supérieure de l'ancre 1, comme illustré aux figures 1a à 2b.

Les premier 2a et second 2b bras portent respectivement une première 4a et une seconde 4b branches s'étendant à l'extérieur de ladite première fente 3a.

La première branche 4a est agencée de manière à définir avec le bras 2a qui la porte une seconde fente 3b. De même, la seconde branche 4b est agencée de manière à définir avec le bras 2b qui la porte une troisième fente 3c.

L'ancre de suture 1 est typiquement monobloc.

Dans sa position de repos, c'est-à-dire dans la position dans laquelle aucune force extérieure ne s'exerce sur elle, l'ancre 1 est dans une position dite « déployée ». Cette position de repos est représentée aux figures 1a, 2a et 3a.

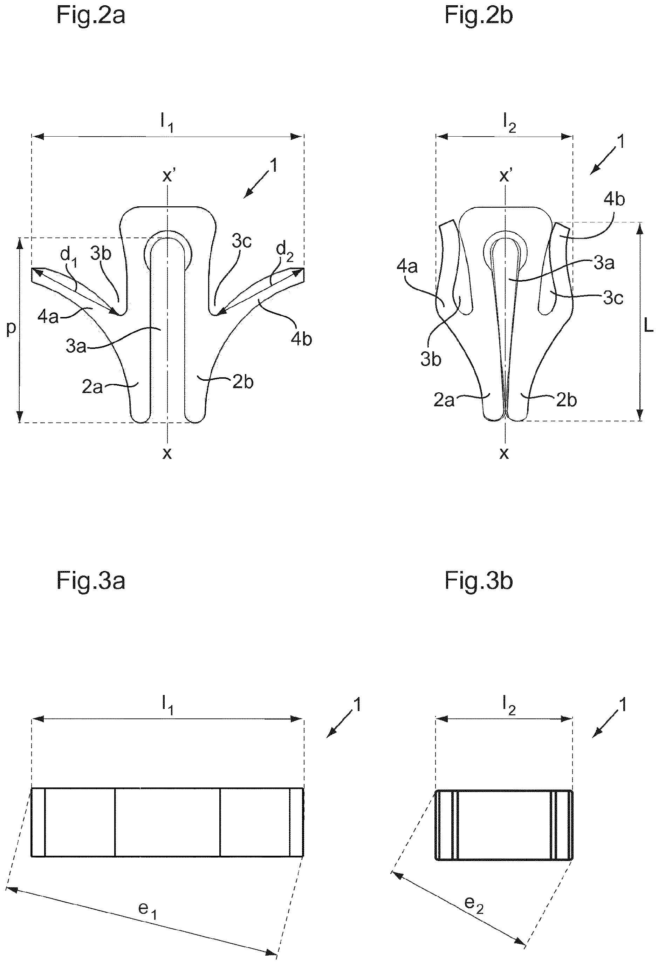

Dans cette position de repos, l'envergure (maximale ou hors-tout) de l'ancre 1, perpendiculairement à l'axe x-x', cette envergure correspondant à l'envergure de l'ensemble comprenant lesdits premier 2a et second 2b bras et lesdites première 4a et seconde 4b branches perpendiculairement à l'axe x-x', correspond à une valeur e1, illustrée à la figure 3a.

A partir de cette position de repos, cette envergure peut être réduite d'une part en rapprochant les deux bras 2a, 2b l'un de l'autre par déformation élastique et d'autre part en rapprochant chacune des branches 4a, 4b du bras 2a, 2b qui la porte et donc de l'axe x-x', comme illustré aux figures 1b, 2b et 3b.

On appellera « position repliée extrême » la position dans laquelle l'envergure (maximale ou hors tout) de l'ancre 1 perpendiculairement à l'axe x-x' est minimale. Dans cette position, les extrémités des deux bras 2a, 2b se touchent de sorte que le contour de la première fente 3a est fermé et chacune des branches 4a, 4b est repliée au maximum contre le bras 2a, 2b qui la porte. Cette position est illustrée aux figures 1b, 2b et 3b et correspond à la position idéale d'insertion de l'ancre 1 dans une phalange P3 d'un doigt. Dans cette position, l'envergure de l'ancre 1 perpendiculairement à l'axe x-x', cette envergure correspondant à l'envergure de l'ensemble comprenant lesdits premier 2a et second 2b bras et lesdites première 4a et seconde 4b branches perpendiculairement à l'axe x-x', correspond à une valeur e2<e1, illustrée à la figure 3b. Le coefficient de réduction k de cette envergure correspond au ratio e2/e1 et est typiquement inférieur ou égal à 0,7.

L'ancre 1 possède une envergure e1 au repos de 4,1mm et une envergure e2 en position repliée extrême de 2mm ; le coefficient de réduction est donc k=0,49. Un faible coefficient de réduction traduit un potentiel de déploiement important. De telles propriétés sont rendues possibles grâce à l'utilisation d'un matériau superélastique pour la fabrication de l'ancre 1. L'ancre 1 est typiquement réalisée en un alliage 55%nickel/45%titane.

L'envergure e1 est légèrement supérieure à la largeur I1=4mm de l'ancre 1 en position déployée (figures 2a et 3a). De même, l'envergure e2 est légèrement supérieure à la largeur I2=1,9mm de l'ancre 1 en position repliée extrême (figures 2b et 3b).

Les bras 2a, 2b et branches 4a, 4b de l'ancre 1 sont agencés de sorte que la projection orthogonale sur l'axe x-x' du fond de chacune des seconde 3b et troisième 3c fentes se situe dans ladite première fente 3a à une distance du fond de cette fente d'au moins 10%, de préférence d'au moins 20%, de préférence d'au moins 30%, de préférence d'au moins 40%, de préférence encore d'au moins 50% de la profondeur p. Dans l'exemple illustré aux figures 1a à 5b, la projection orthogonale sur l'axe x-x' du fond de chacune des seconde 3b et troisième 3c fentes se situe dans ladite première fente 3a à une distance du fond de cette fente de 43% environ.

En outre, les bras 2a, 2b et branches 4a, 4b de l'ancre 1 sont disposés de sorte que, en position de repos, la projection orthogonale sur l'axe x-x' de chacune desdites première 4a et seconde 4b branches se situe complètement dans ladite première fente 3a, comme illustré à la figure 2a. En variante, il serait possible que seule une partie de la longueur de la projection orthogonale de chacune des branches 4a, 4b sur l'axe x-x', typiquement au moins 70% de cette longueur, se situe dans la première fente 3a en position de repos.

La longueur des branches 4a, 4b relativement à la profondeur p est importante. En référence à la figure 2a, soit d1 la distance séparant le fond de la seconde fente 3b de la partie de l'extrémité de la branche 4a qui la définie qui en est la plus éloignée et soit d2 la distance séparant le fond de la troisième fente 3c de la partie de l'extrémité de la branche qui la définie qui en est la plus éloignée, lorsque l'ancre 1 est en position de repos. L'ancre de suture 1 est conçue de sorte que chacun des ratios d1/p et d2/p soit supérieur ou égal à 0,3. Dans l'ancre 1 illustrée dans les figures, d1 = d2 = 1,8 mm et p = 2,75 mm. Les ratios d1/p et d2/p sont donc tous les deux environ égaux à 0,65.

L'ancre de suture 1 est destinée à être ancrée dans la phalange distale P3 d'un doigt de la main. Elle doit avoir un potentiel de déploiement le plus important possible ce qui se traduit par un faible ratio e2/e1 c'est-à-dire un faible coefficient de réduction « k ».

Les proportions relatives des valeurs d1, d2 et p définies précédemment participent à l'obtention d'un ratio avantageux entre l'envergure perpendiculairement à l'axe x-x' en position de repos (e1) et l'envergure perpendiculairement à l'axe x-x' en position repliée extrême (e2).

Les surfaces avant et arrière de l'ancre de suture 1 sont typiquement planes et parallèles à un même plan P₁ incluant l'axe x-x'. L'ancre de suture 1 présente typiquement un plan de symétrie orthogonale P₂ normal au plan P₁ et incluant également l'axe x-x". Ces plans P₁ et P₂ sont illustrés à la figure 1a.

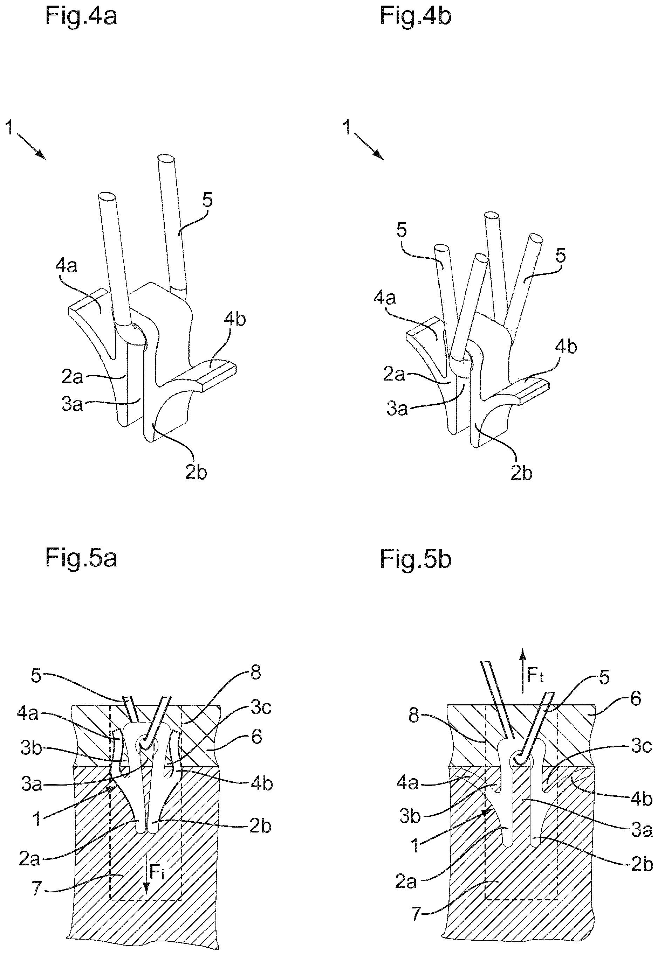

Pour venir refixer les tendons ou les ligaments endommagés à leur place sur la phalange P3, l'ancre de suture 1 est typiquement ancrée dans cette phalange, sous l'os cortical. Elle porte un ou plusieurs fils 5 de suture pour suturer le tissu, tendon ou ligament, endommagé. La figure 4a illustre l'ancre 1 en position déployée avec un fil de suture en position dans sa première fente 3a. Etant donné que la première fente 3a possède un contour ouvert en position de repos, le chirurgien peut, dans cette position, choisir et positionner lui-même le fil de suture 5 dont il a besoin et cela sans passe-fil. La première fente 3a est en outre suffisamment profonde pour pouvoir accueillir plusieurs fils de suture 5 si nécessaire, comme illustré à la figure 4b.

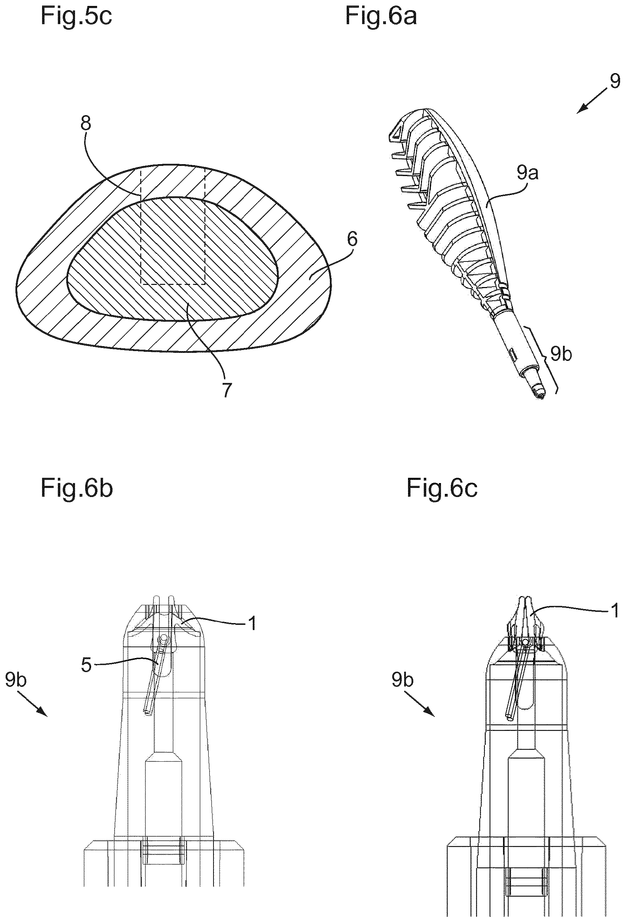

En pratique, l'ancre de suture 1 est destinée à être insérée dans un trou 8 traversant de part en part la partie corticale 6 dorsale de la phalange P3 et s'étendant en partie dans l'os spongieux 7, comme illustré aux figures 5a, 5b et 5c.

Le trou 8 est typiquement un perçage cylindrique dont le diamètre est le plus petit diamètre autorisant le passage de l'ancre 1 lorsque celle-ci est en position repliée extrême. Ce diamètre est typiquement égal à l'envergure maximale e2 de l'ancre 1 en position repliée extrême, soit 2mm. Il s'agit de la plus grande longueur séparant deux parties de l'ancre 1 en position repliée extrême, perpendiculairement à l'axe x-x' (en vue de dessus). Le diamètre de ce perçage est également suffisamment étroit pour empêcher l'ancre 1 de ressortir du trou 8 une fois déployée dans l'os.

L'ancre de suture 1 est destinée à être insérée avec sa partie inférieure (côté x de l'axe x-x') en avant dans le sens d'une force d'insertion Fᵢ orientée dans le prolongement du trou 8, c'est-à-dire suivant l'axe de révolution du perçage cylindrique, comme illustré à la figure 5a. Ses branches 4a, 4b sont destinées à traverser complètement l'épaisseur de l'os cortical 6 puis à se déployer dans l'os spongieux 7 sous l'effet des forces de rappel élastiques s'exerçant sur les bras 2a, 2b et sur les branches 4a, 4b et tendant à les ramener dans leur position de repos, comme illustré à la figure 5b.

Pour permettre un ancrage solide sans risquer d'endommager l'os cortical, l'ancre 1 doit être conçue de sorte que ses bras et branches soient aptes à se déployer suffisamment, à partir de sa position repliée d'insertion, idéalement presque complètement voire complètement, tant qu'elle est dans l'os spongieux 7 mais pour qu'ils soient inaptes à se déployer, même partiellement, lorsqu'elle est dans l'os cortical 6.

Dans le cadre de l'invention, on considère que l'ensemble comprenant les bras 2a, 2b et branches 4a, 4b de l'ancre 1 est apte à se déployer dans l'os spongieux 7 si cet ensemble est apte à se déployer d'une première position correspondant à sa position repliée d'insertion jusqu'à une seconde position suffisamment déployée lorsqu'il est noyé dans n'importe quel matériau dont la pression à la rupture en compression est inférieure ou égale à 10 MPa, de préférence inférieure ou égale à 15MPa.

On entend par « position repliée d'insertion », une position dans laquelle l'envergure de l'ensemble comprenant les bras 2a, 2b et branches 4a, 4b de l'ancre 1 perpendiculairement à l'axe x-x' est diminuée d'au moins 30%, de préférence d'au moins 40%, de préférence encore d'au moins 45% par rapport à son envergure perpendiculairement à l'axe x-x' en position de repos, et par « position suffisamment déployée » une position dans laquelle l'envergure de cet ensemble perpendiculairement à l'axe x-x' est égale à au moins 75%, de préférence à au moins 80%, de préférence à au moins 85%, de préférence à au moins 90%, de préférence encore à au moins 95% voire à 100% de son envergure perpendiculairement à l'axe x-x' dans sa position déployée (de repos).

Dans le cadre de l'invention, on considère également que l'ancre 1 ne peut pas se déployer même partiellement dans l'os cortical 6 si l'ensemble comprenant ses bras 2a, 2b et branches 4a, 4b est incapable de se déployer, même partiellement, lorsqu'il est noyé dans n'importe quel matériau dont la pression à la rupture en compression est supérieure ou égale à 200 MPa, de préférence supérieure ou égale à 100MPa, de préférence supérieure ou égale à 50 MPa.

Une fois l'ancre 1 en place dans l'os et la suture réalisée, le fil de suture 5 exerce une force de traction Fₜ sur l'ancre 1. Les branches 4a, 4b se déploient dans l'os spongieux 7 et les parties des branches 4a, 4b qui, en position de repos, sont orientées en regard de l'axe x-x' viennent typiquement en appui contre l'intérieur de la partie corticale 6 de la phalange P3, empêchant ainsi l'ancre 1 d'en ressortir. Cela est illustré à la figure 5b.

Pour insérer l'ancre de suture 1 dans le trou 8, le chirurgien utilise typiquement une pince ou un instrument 9 tel que représenté à la figure 6a.

Un tel instrument 9 comprend un manche 9a et une partie active 9b. La partie active 9b comprend un logement dans lequel l'ancre 1 peut être positionnée et maintenue en position déployée (cf. figure 6b). Un poussoir permet d'exercer une force sur la partie supérieure de l'ancre 1 pour la replier, typiquement jusqu'à sa position repliée extrême, en l'introduisant à force dans une ouverture aux dimensions adaptées (cf. figure 6c).

L'ancre de suture 1 est particulièrement adaptée pour être utilisée dans une phalange distale P3 d'un doigt de la main mais peut être utilisée dans d'autres os, ses dimensions pouvant être adaptées, le cas échéant.

Une méthode pour la mise en place d'une ancre de suture orthopédique 1 telle que définie précédemment comprend typiquement les étapes suivantes:
- réaliser un perçage 8 traversant de part en part la partie corticale 6 d'un os et s'étendant dans la partie spongieuse 7 dudit os ;
- positionner un ou plusieurs fils de suture 5 dans la première fente 3a de ladite ancre de suture 1, cela pouvant typiquement être effectué par le chirurgien, par exemple avec l'ancre de suture 1 maintenue en position déployée dans un instrument tel que l'instrument 9 décrit précédemment ;
- amener l'ancre de suture 1 dans une position repliée d'insertion c'est-à-dire diminuer l'envergure de l'ensemble comprenant ses premier 2a et second 2b bras et ses première 4a et seconde 4b branches perpendiculairement à l'axe x-x' d'au moins 30% par rapport à ladite envergure en position de repos, d'une part par le rapprochement élastique de chacune des branches 2a, 2b du bras 4a, 4b qui la porte et d'autre part par le rapprochement élastique desdits premier 2a et second 2b bras l'un de l'autre, cela pouvant typiquement être effectué par le chirurgien en exerçant un pression sur les branches 4a, 4b à l'aide d'une pince ou à l'aide d'un instrument tel que l'instrument 9 décrit précédemment ;
- insérer l'ensemble comprenant lesdits premier 2a et second 2b bras et lesdites première 4a et seconde 4b branches à travers ce perçage 8 en le maintenant dans ladite position repliée d'insertion ;
- arrêter de maintenir l'envergure dudit ensemble dans ladite position repliée d'insertion, typiquement en relâchant la pression exercée sur ses branches 4a, 4b de sorte que ledit ensemble se déploie au moins partiellement dans la partie spongieuse 7 dudit os ; et
- suturer un tissu endommagé tel qu'un ligament ou un tendon avec le ou les fils de suture 5.

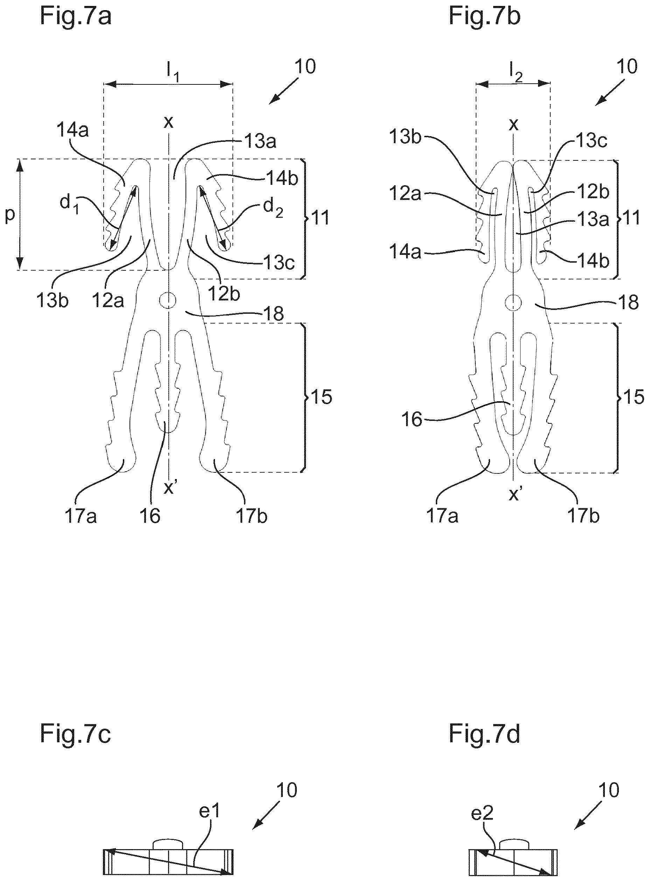

En référence aux figures 7a à 10, un dispositif d'ancrage osseux selon un second mode de réalisation de l'invention est un implant 10 pour arthrodèse typiquement pour une articulation inter-phalangienne distale.

L'implant 10 comprend une première partie d'ancrage 11 destinée à être ancrée dans une phalange distale P3 d'un doigt de la main et une seconde partie d'ancrage 15 destinée à être ancrée dans la phalange intermédiaire P2 biologiquement articulée avec ladite phalange distale P3 de manière à former un pont osseux entre ces deux phalanges.

Ces première 11 et seconde 15 parties d'ancrage sont reliées par l'intermédiaire d'une portion rigide centrale 18.

La première partie d'ancrage 11 possède une structure très similaire à celle de l'ancre de suture 1 selon le premier mode de réalisation de l'invention. Elle comprend un premier 12a et un second 12b bras ainsi que des première 14a et seconde 14b branches.

Les premier 12a et second 12b bras sont typiquement agencés en forme de U ou de V et définissent entre eux une première fente 13a. Ils portent respectivement lesdites première 14a et seconde 14b branches qui s'étendent à l'extérieur de ladite première fente 13a.

Cette première fente 13a a une profondeur de longueur p et s'étend selon un axe x-x', x correspondant à la partie supérieure et x' à la partie inférieure de l'implant 10, comme illustré aux figures 7a et 7b.

La première branche 14a est agencée de manière à définir avec le bras 12a qui la porte une seconde fente 13b. De même, la seconde branche 14b est agencée de manière à définir avec le bras 12b qui la porte une troisième fente 13c.

L'implant 10 est typiquement monobloc.

Dans sa position de repos, c'est-à-dire dans la position dans laquelle aucune force extérieure ne s'exerce sur elle, la première partie d'ancrage 11 de l'implant 10 est dans une position dite « déployée ». Cette position de repos est représentée à la figure 7a. Dans cette position de repos, l'envergure (maximale ou hors-tout) de la première partie d'ancrage 11, perpendiculairement à l'axe x-x', cette envergure correspondant à l'envergure de l'ensemble comprenant lesdits premier 12a et second 12b bras et lesdites première 14a et seconde 14b branches perpendiculairement à l'axe x-x', correspond à une valeur e1, illustrée à la figure 7c.

A partir de cette position de repos, cette envergure peut être réduite d'une part en rapprochant les deux bras 12a, 12b l'un de l'autre par déformation élastique et d'autre part en rapprochant chacune des branches 14a, 14b du bras 12a, 12b qui la porte et donc de l'axe x-x', comme illustré à la figure 7b.

Comme pour l'ancre de suture 1, on appelle « position repliée extrême » la position dans laquelle l'envergure (maximale ou hors tout) de la première partie d'ancrage 11 de l'implant 10 perpendiculairement à l'axe x-x' est minimale. Dans cette position, les extrémités des deux bras 12a, 12b se touchent de sorte que le contour de la première fente 13a est fermé et chacune des branches 14a, 14b est repliée au maximum contre le bras 12a, 12b qui la porte. Cette position est illustrée à la figure 7b et correspond à la position d'insertion idéale de la première partie d'ancrage 11 de l'implant 10 dans une phalange distale P3 d'un doigt. Dans cette position, l'envergure de la première partie d'ancrage 11 perpendiculairement à l'axe x-x' correspond à une valeur e2<e1, illustrée à la figure 7d. Le coefficient de réduction k de cette envergure correspond au ratio e2/e1 et est typiquement inférieur ou égal à 0,7.

La première partie d'ancrage 11 de l'implant 10 illustré dans les figures possède une envergure e1 au repos de 6,1mm et une envergure e2 en position repliée extrême de 3,3mm, le coefficient de réduction est donc k=0,54 environ. Un faible coefficient de réduction traduit un potentiel de déploiement important. De telles propriétés sont rendues possibles grâce à l'utilisation d'un matériau superélastique tel qu'un alliage 55%nickel/45%titane pour la fabrication de l'implant 10.

L'envergure e1 est légèrement supérieure à la largeur I1=6mm de la première partie d'ancrage 11 en position déployée (figures 7a et 7c). De même, l'envergure e2 est légèrement supérieure à la largeur I2=3,1 mm de la première partie d'ancrage 11 en position repliée extrême (figures 7b et 7d).

Les bras 12a, 12b et branches 14a, 14b de la première partie d'ancrage 11 de l'implant 10 sont agencés de sorte que la projection orthogonale sur l'axe x-x' du fond de chacune des seconde 13b et troisième 13c fentes se situe dans ladite première fente 13a à une distance du fond de cette fente d'au moins 10%, de préférence d'au moins 20%, de préférence d'au moins 30%, de préférence d'au moins 40%, de préférence d'au moins 50%, de préférence encore d'au moins 75% de la profondeur p. Dans l'exemple illustré aux figures 7a à 10, la projection orthogonale sur l'axe x-x' du fond de chacune des seconde 3b et troisième 3c fentes se situe dans ladite première fente 3a à une distance du fond de cette fente de 84% environ.

En outre, les bras 12a, 12b et branches 14a, 14b de la première partie d'ancrage 11 de l'implant 10 sont disposés de sorte que, en position de repos, la projection orthogonale sur l'axe x-x' de chacune desdites première 14a et seconde 14b branches se situe complètement dans ladite première fente 13a (cf. figure 7a). En variante, il serait possible que seule une partie de la longueur de la projection orthogonale de chacune des branches 14a, 14b sur l'axe x-x', typiquement au moins 70% de cette longueur, se situe dans la première fente 13a en position de repos.

Comme pour l'ancre de suture 1, la longueur des branches 14a, 14b de l'implant 10 relativement à la profondeur p est importante. En référence à la figure 7a, soit d1 la distance séparant le fond de la seconde fente 13b de la partie de l'extrémité de la branche 14a qui la définie qui en est la plus éloignée et soit d2 la distance séparant le fond de la troisième fente 13c de la partie de l'extrémité de la branche 14b qui la définie qui en est la plus éloignée, lorsque la première partie d'ancrage 11 est en position de repos. L'implant 10 est conçu de sorte que chacun des ratios d1/p et d2/p soit supérieur ou égal à 0,3. En ce qui concerne la première partie d'ancrage 11 de l'implant 10, d1 = d2 = 3,45 mm et p = 5,5 mm de sorte que d1/p et d2/p sont tous les deux environ égaux à 0,63.

Comme indiqué précédemment, la première partie d'ancrage 11 de l'implant 10 est destinée à être ancrée dans la phalange distale P3 d'un doigt de la main. Etant données les petites dimensions d'un tel os, le perçage pour l'insertion de la partie d'ancrage 11 dans la phalange P3 doit avoir un diamètre le plus petit possible. En effet, préserver l'os est important pour la résistance mécanique de la phalange P3 ainsi que pour maximiser la surface d'os en contact entre les phalanges P2 et P3 en vue de leur fusion osseuse. Malgré cette contrainte, l'implant 10 doit permettre un ancrage le plus résistant possible. La première partie d'ancrage 11 doit donc avoir un potentiel de déploiement important ce qui se traduit par un faible ratio e2/e1 et donc par un faible coefficient de réduction k.

Les proportions relatives des valeurs d1, d2 et p définies précédemment participent à l'obtention d'un ratio avantageux entre l'envergure perpendiculairement à l'axe x-x' en position de repos (e1) et l'envergure perpendiculairement à l'axe x-x' en position repliée extrême (e2).

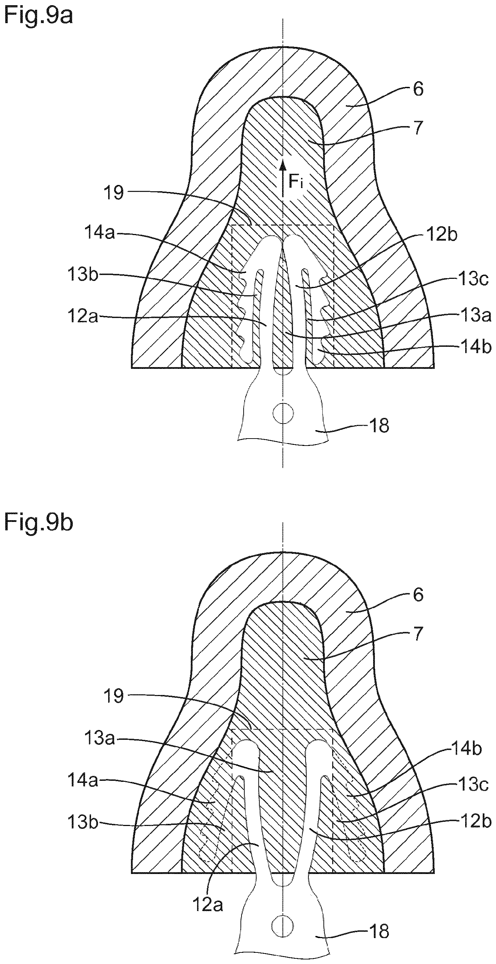

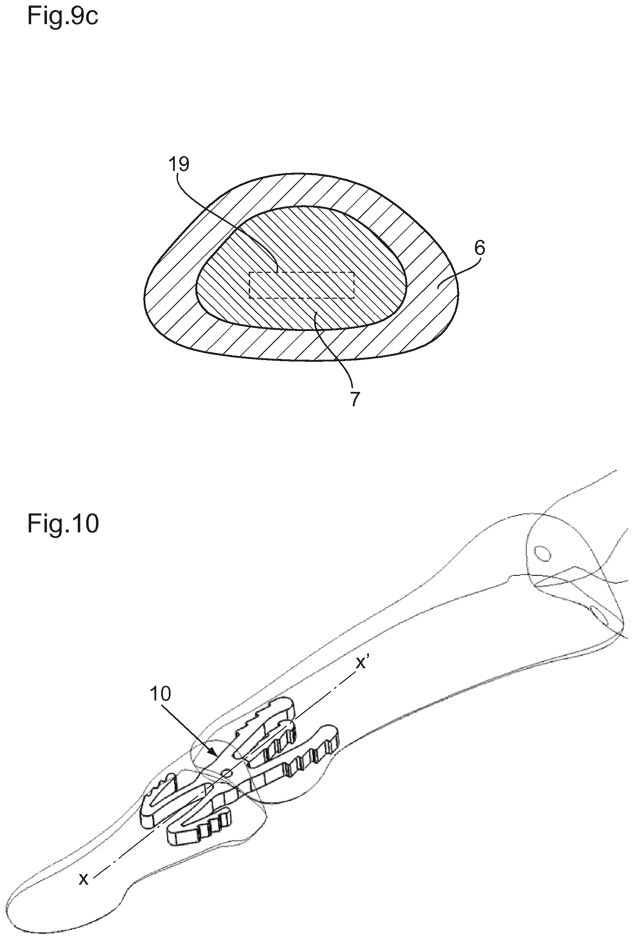

En pratique, la première partie d'ancrage 11 sera insérée dans un premier trou 19 s'étendant dans la partie d'os spongieux 7 de la phalange P3, comme illustré aux figures 9a, 9b et 9c.

Pour réaliser un tel trou 19, la phalange P3 est avivée au niveau de sa jonction avec la phalange P2 puis le trou 19 est réalisé dans l'os spongieux 7. La figure 9a illustre une coupe longitudinale de la phalange P3 après avivage et réalisation du trou 19. Le trou 19 est typiquement un trou borgne calibré, réalisé à l'aide d'une râpe spéciale. Ses dimensions sont adaptées aux dimensions de la première partie d'ancrage 11.

La première partie d'ancrage 11 est destinée à être insérée avec sa partie supérieure (côté x' de l'axe x-x') en avant dans le sens d'une force d'insertion Fi orientée dans le prolongement du premier trou 19, comme illustré à la figure 9a. Les branches 14a, 14b sont destinées à s'insérer entièrement dans ledit premier trou 8 puis à se déployer dans l'os spongieux 7 sous l'effet des forces de rappel élastique s'exerçant sur les bras 12a, 12b et branches 14a, 14b et tendant à les ramener dans leur position de repos, comme illustré à la figure 9b.

Pour permettre un ancrage solide sans risquer d'endommager l'os cortical 6, la première partie d'ancrage 11 doit être conçue de sorte que ses bras 12a, 12b et branches 14a, 14b soient aptes à se déployer suffisamment, à partir de sa position repliée d'insertion, idéalement presque complètement voire complètement, tant qu'elle est dans l'os spongieux 7 mais pour qu'ils soient inaptes à se déployer, même partiellement, lorsqu'elle est dans l'os cortical 6.

Comme pour le premier mode de réalisation de l'invention, on considère que l'ensemble comprenant les bras 12a, 12b et branches 14a, 14b de la première partie d'ancrage 11 de l'implant 10 est capable de se déployer dans l'os spongieux 7 si cet ensemble est apte à se déployer d'une première position correspondant à sa position repliée d'insertion jusqu'à une seconde position suffisamment déployée lorsqu'il est noyé dans n'importe quel matériau dont la pression à la rupture en compression est inférieure ou égale à 10MPa, de préférence inférieure ou égale à 15MPa.

On entend par « position repliée d'insertion », une position dans laquelle l'envergure de l'ensemble comprenant les bras 12a, 12b et branches 14a, 14b de la première partie d'ancrage 11 perpendiculairement à l'axe x-x' est diminuée d'au moins 30%, de préférence d'au moins 40%, de préférence encore d'au moins 45% par rapport à son envergure perpendiculairement à l'axe x-x' en position de repos, et par « position suffisamment déployée » une position dans laquelle l'envergure de cet ensemble perpendiculairement à l'axe x-x' est égale à au moins 75%, de préférence à au moins 80%, de préférence à au moins 85%, de préférence à au moins 90%, de préférence encore à au moins 95% voire à 100% de son envergure perpendiculairement à l'axe x-x' dans sa position déployée (de repos).

Dans le cadre de l'invention, on considère également que la première partie d'ancrage 11 de l'implant 10 ne peut pas se déployer même partiellement dans l'os cortical 6 si l'ensemble comprenant ses bras 12a, 12b et branches 14a, 14b est incapable de se déployer, même partiellement, lorsqu'il est noyé dans n'importe quel matériau dont la pression à la rupture en compression est supérieure à 200 MPa, de préférence supérieure ou égale à 100MPa, de préférence supérieure ou égale à 50 MPa.

De par la structure de ses bras 12a, 12b et de ses branches 14a, 14b, la première partie d'ancrage 11 de l'implant 10 est en forme de sablier. Cette forme est particulièrement adaptée à la forme de la phalange distale P3 des doigts de la main, comme illustré à la figure 10. De part cette forme, les côtés des branches 14a, 14b orientés à l'opposé de l'axe x-x' peuvent venir en appui contre l'intérieur de la partie corticale 6 de la phalange en créant des zones d'appui non ponctuelles, typiquement réparties sur l'ensemble de la longueur des branches 14a, 14b, ce qui évite d'endommager l'os. Les branches 14a, 14b sont typiquement pourvues de crans pour améliorer leur ancrage dans la phalange P3.

Les surfaces avant et arrière des premier 12a et second 12b bras et des première 14a et seconde 14b branches de la première partie d'ancrage 11 sont planes et parallèles à un même premier plan incluant l'axe x-x'.

L'implant 10 étant un implant intramédullaire pour arthrodèse IPD, sa seconde partie d'ancrage 15 a une forme différente de la première partie d'ancrage 11, cette forme étant particulièrement adaptée à la forme des phalanges P2.

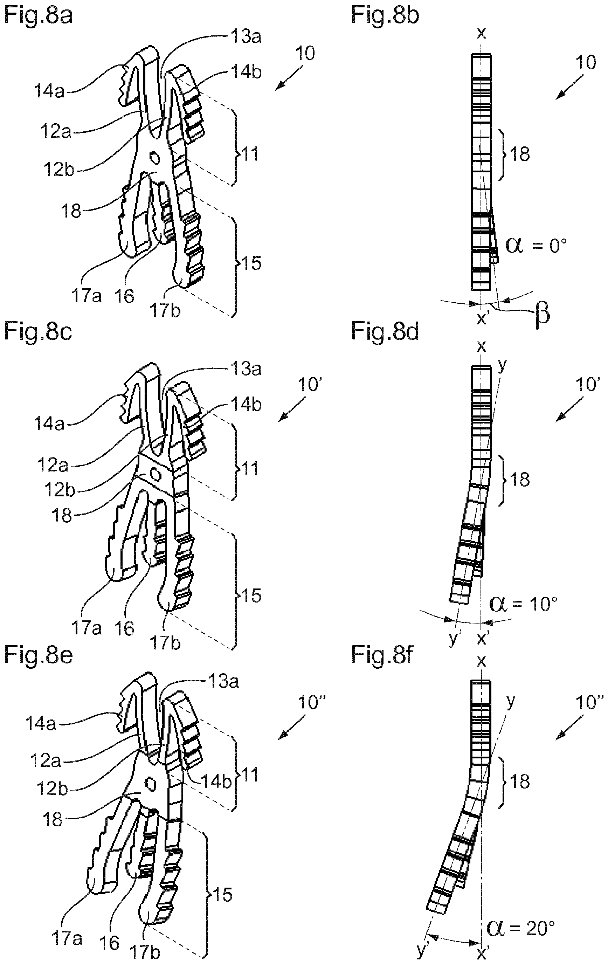

La seconde partie d'ancrage 15 comprend typiquement trois bras 16, 17a, 17b agencés de sorte que, en position de repos, deux 17a, 17b desdits bras appelés bras principaux définissent l'envergure (maximale ou hors tout) de ladite seconde partie d'ancrage 15 perpendiculairement à un axe y-y'. Dans l'implant 10 représenté aux figure 7a à 8b, l'axe y-y' est confondu avec l'axe x-x'.

Pour la seconde partie d'ancrage 15 également, il est important que son envergure perpendiculairement à l'axe y-y' soit plus faible à l'état replié extrême qu'à l'état de repos, c'est-à-dire qu'elle possède un potentiel de déploiement important entre la position repliée dans laquelle elle est insérée dans l'os et la position qu'elle prendra une fois déployée.

La seconde partie d'ancrage 15 s'étend majoritairement dans un second plan incluant l'axe y-y'.

En variante, l'axe y-y' peut former un angle α avec l'axe x-x', α étant typiquement compris entre 0° et 40°, de préférence entre 0° et 25°, de préférence encore entre 0° et 20°. Dans l'exemple illustré aux figures 7a à 8b, α=0°.

Les figures 8c à 8f illustrent deux variantes d'implants appartenant à une gamme d'implants selon l'invention. Dans une première de ces variantes (implant 10' illustré aux figures 8c et 8d), l'angle α est de 10°. Dans une seconde de ces variantes (implant 10" illustré aux figures 8e et 8f), l'angle α est de 20°.

Les variantes d'implants 10, 10', 10" forment une gamme d'implants dans laquelle le chirurgien pourra choisir afin d'adapter la chirurgie en fonction de l'articulation touchée et du désir du patient. Cela est particulièrement important dans le cas d'une arthrodèse sur une articulation IPD. En effet, l'angle entre les phalanges distale et médiane est capital dans la fonction de préhension de la main.

C'est la portion rigide centrale 18 de chacun des implants 10, 10', 10" qui détermine l'angle α entre lesdits premier et le second plans

Avantageusement, la portion rigide centrale 18 de l'implant 10 comprend un perçage central. Celui-ci permet un maintien en position de l'implant 10, 10', 10" via une broche de kirschner pendant l'opération, cette broche étant retirée à la fin de l'opération.

Le troisième 16 des bras de la seconde partie d'ancrage 15, appelé bras additionnel, s'étend dans un troisième plan formant un angle β de 10° environ avec ledit second plan. Lesdits trois bras 16, 17a, 17b forment un trépied. Cela est visible à la figure 8a. En variante, l'angle β peut varier. Il est typiquement compris entre 7° et 15°, de préférence entre 10° et 15°.

Les bras principaux 17a, 17b de la seconde partie d'ancrage 15 sont typiquement pourvus de crans. Le bras additionnel 16 de la seconde partie d'ancrage 15 est de préférence également pourvu de crans. L'ensemble de ces crans vise à améliorer l'ancrage desdits bras 16, 17a, 17b dans la phalange P2.

L'agencement en forme de trépied des trois bras 16, 17a, 17b permet un bon ancrage. Un tel agencement est particulièrement adapté pour une phalange médiane P2 d'un doigt la main. Il apporte une stabilité dorso-palmaire à l'implant 10, 10', 10" et évite les effondrements de la corticale dorsale.

En pratique, la seconde partie d'ancrage 15 est insérée dans un second trou réalisé dans la partie corticale de la phalange P2.

Comme le premier trou 19, ce second trou est un trou borgne calibré, typiquement réalisé à l'aide d'une râpe spéciale après avivage de la phalange P2, et dont les dimensions sont adaptées aux dimensions de la seconde partie d'ancrage 15.

La caractéristique principale des implants d'arthrodèse 10, 10', 10" décrits ci-dessus réside dans le fait que leur première partie d'ancrage 11 présente une double expansion, à la fois de ses bras 12a, 12b et de ses branches 14a, 14b.

Dans chacun des implants d'arthrodèse 10, 10', 10" illustrés aux figures 7a à 10, les première 11 et seconde 15 parties d'ancrage sont différentes l'une de l'autre, chacune possédant une forme particulièrement adaptée à la phalange avec laquelle elle va coopérer pour l'arthrodèse IPD. Cependant, dans la mesure où de tels implants 10, 10', 10" pourraient être utilisés dans d'autres os, l'homme du métier pourrait adapter les formes et dimensions des parties d'ancrages 11, 15 en fonction des os pour lesquels l'arthrodèse serait envisagée. Elles pourraient par exemple être toutes les deux identiques à la première partie d'ancrage 11.

Une méthode pour la mise en place d'un implant 10, 10', 10" tel que défini précédemment comprend typiquement les étapes suivantes :
i. réaliser un premier trou 19 calibré dans la partie spongieuse 7 d'un premier os d'une articulation entre deux os à traiter et un second trou calibré dans la partie spongieuse du second os de ladite articulation ;
ii. ancrer la seconde partie d'ancrage 15 de l'implant 10 dans ledit second trou en réalisant les étapes suivantes :
   o amener la seconde partie d'ancrage 15 de l'implant 10 dans une position repliée d'insertion, c'est-à-dire diminuer l'envergure de ladite seconde partie d'ancrage 15 perpendiculairement à l'axe y-y', cela pouvant typiquement être effectué par le chirurgien en exerçant un pression sur les bras principaux 17a, 17b par exemple à l'aide d'une pince ou à l'aide d'un instrument d'insertion ;
   o insérer la seconde partie d'ancrage 15 dans le second trou en maintenant ladite position repliée d'insertion ; et
   o arrêter de maintenir la seconde partie d'ancrage 15 dans ladite position repliée d'insertion, typiquement en relâchant la pression exercée sur les bras 17a, 17b ;
iii. ancrer la première partie d'ancrage 11 de l'implant 10 dans ledit premier trou en réalisant les étapes suivantes :
   o amener la première partie d'ancrage 11 de l'implant 10 dans une position repliée d'insertion, c'est-à-dire diminuer l'envergure de l'ensemble comprenant lesdits premier 12a et second 12b bras et lesdites première 14a et seconde 14b branches perpendiculairement à l'axe x-x' d'au moins 30% par rapport à ladite envergure en position de repos, d'une part par le rapprochement élastique de chacune des branches 14a, 14b du bras 12a, 12b qui la porte et d'autre part par le rapprochement élastique desdits premier 12a et second 12b bras l'un de l'autre, cela pouvant typiquement être effectué par le chirurgien en exerçant un pression sur les branches 14a, 14b par exemple à l'aide d'une pince ;
   o insérer l'ensemble comprenant lesdits premier 12a et second 12b bras et lesdites première 14a et seconde 14b branches de ladite première partie d'ancrage 11 dans ledit premier trou 19 en maintenant ladite position repliée d'insertion ;
   o arrêter de maintenir ledit ensemble dans ladite position repliée d'insertion, typiquement en relâchant la pression exercée sur les branches 14a, 14b, de sorte que ledit ensemble se déploie au moins partiellement dans la partie spongieuse 7 dudit premier os.

En variante, l'étape iii peut être réalisée avant l'étape ii.

De préférence, une étape préliminaire à l'étape i consiste à aviver la surface articulaire de chacun des premier et second os de ladite articulation à traiter.

Il apparaîtra clairement à l'homme du métier que la présente invention n'est en aucun cas limitée aux modes de réalisation présentés ci-dessus et illustrés dans les figures.

Il est évident que la forme des bras et branches de l'ancre ou de la première partie d'ancrage de l'implant selon l'invention peuvent varier à l'infini pour autant que leurs fonctions soient assurées.

Il est également très bien envisageable de réaliser un dispositif d'ancrage osseux selon l'invention autre qu'une ancre de suture ou qu'un implant pour arthrodèse, par exemple une tige d'arthroplastie ou une vis d'interférence.

La présente invention est limitée par les revendications annexées.

Le dispositif d'ancrage osseux selon l'invention présente l'avantage de posséder un fort potentiel de déploiement, c'est-à-dire qu'il possède une position repliée dans laquelle son envergure est très étroite et une position déployée dans laquelle son envergure peut prendre beaucoup d'ampleur. Ainsi, il peut, en position repliée, être inséré dans des trous de diamètres extrêmement petits et permettre cependant un fort ancrage en se déployant dans l'os. Typiquement, pour une envergure donnée à l'état replié extrême on obtient un déploiement de l'ensemble comprenant les bras et branches de la partie d'ancrage bien plus important que dans les dispositifs d'ancrage selon l'art antérieur. Cela représente un grand intérêt, en particulier pour les dispositifs d'ancrage osseux utilisés dans de petits os dont les dimensions et la fragilité limitent la taille du perçage.

## Revendications

1. Dispositif d'ancrage osseux (1 ; 10) comprenant un premier (2a ; 12a) et un second (2b ; 12b) bras définissant entre eux une première fente (3a ; 13a) de profondeur p s'étendant selon un axe x-x', lesdits premier (2a ; 12a) et second (2b ; 12b) bras portant respectivement une première (4a ; 14a) et une seconde (4b ; 14b) branche s'étendant à l'extérieur de ladite première fente (3a ; 13a), ladite première branche (4a ; 14a) étant agencée de manière à définir avec le bras (2a, 12a) qui la porte une seconde fente (3b ; 13b) et ladite seconde branche (4b ; 14b) étant agencée de manière à définir avec le bras (2b ; 12b) qui la porte une troisième fente (3c ; 13c), **caractérisé par le fait que** ledit dispositif (1 ; 10) est agencé de sorte que, en position de repos :
- la projection orthogonale sur l'axe (x-x') du fond de chacune des seconde (3b ; 13b) et troisième (3c ; 13c) fentes se situe dans ladite première fente (3a ; 13a) à une distance du fond de cette fente d'au moins 10%, de préférence d'au moins 20%, de préférence d'au moins 30%, de préférence d'au moins 40%, de préférence encore d'au moins 50% de la profondeur p ; et
- les distances d1 séparant le fond de ladite seconde fente (3b ; 13b) de la partie de l'extrémité de la branche (4a ; 14a) qui la définit qui en est la plus éloignée et d2 séparant le fond de ladite troisième fente (3c ; 13c) de la partie de l'extrémité de la branche (4b ; 14b) qui la définit qui en est la plus éloignée soient telles que chacun des ratios d1/p et d2/p soit supérieur ou égal à 0,3, de préférence supérieur ou égal à 0,4, de préférence encore supérieur ou égal à 0,5 ;
ledit dispositif (1 ; 10) étant apte à diminuer l'envergure de l'ensemble comprenant lesdits premier (2a ; 12a) et second (2b ; 12b) bras et lesdites première (4a ; 14a) et seconde (4b ; 14b) branches perpendiculairement à l'axe (x-x') à partir de ladite position de repos, d'une part par le rapprochement élastique de chacune des branches (4a, 4b ; 14a, 14b) du bras (2a, 2b ; 12a, 12b) qui la porte et d'autre part par le rapprochement élastique desdits premier (2a ; 12a) et second (2b ; 12b) bras l'un de l'autre, pour son insertion, au moins en partie, dans un os.

2. Dispositif d'ancrage osseux (1 ; 10) selon la revendication 1, **caractérisé en ce que** l'envergure de l'ensemble comprenant lesdits premier (2a ; 12a) et second (2b ; 12b) bras et lesdites première (4a ; 14a) et seconde (4b ; 14b) branches perpendiculairement à l'axe x-x' peut, lors desdits rapprochements élastiques, être réduite d'au moins 30% par rapport à ladite envergure en position de repos.

3. Dispositif d'ancrage osseux (1 ; 10) selon l'une des revendications précédentes, **caractérisé en ce qu'**il est conçu de sorte que l'ensemble comprenant ses bras (2a, 2b ; 12a, 12b) et branches (4a, 4b ; 14a, 14b) soit apte à se déployer d'une première position repliée dans laquelle son envergure perpendiculairement à l'axe x-x' est inférieure ou égale à 0,7, de préférence inférieure ou égale à 0,6, de préférence encore inférieure ou égale à 0,55 fois son envergure perpendiculairement à l'axe x-x' en position de repos, jusqu'à une seconde position dans laquelle son envergure perpendiculairement à l'axe x-x' est supérieure ou égale à 0,75, de préférence 0,80, de préférence 0,85, de préférence 0,90, de préférence 0,95 fois son envergure perpendiculairement à l'axe x-x' en position de repos, de préférence encore jusqu'à sa position de repos, dans les matériaux dont la pression à la rupture en compression est inférieure ou égale à 10 MPa, de préférence inférieure ou égale à 15MPa, tout en étant incapable de se déployer même partiellement dans les matériaux dont la pression à la rupture en compression est supérieure ou égale à 200 MPa, de préférence supérieure ou égale à 100MPa, de préférence supérieure ou égale à 50 MPa.

4. Dispositif d'ancrage osseux (1 ; 10) selon l'une des revendications précédentes, **caractérisé en ce qu'**il est réalisé en un matériau superélastique tel qu'un alliage nickel/titane.

5. Dispositif d'ancrage osseux (1 ; 10) selon l'une des revendications précédentes, **caractérisé en ce que**, dans ladite position de repos, la projection orthogonale sur l'axe x-x' de chacune desdites première (4a ; 14a) et seconde (4b ; 14b) branches se situe complètement ou presque complètement dans ladite première fente (3a ; 13a).

6. Dispositif d'ancrage osseux (1 ; 10) selon l'une des revendications précédentes, **caractérisé en ce qu'**il est monobloc.

7. Dispositif d'ancrage osseux selon l'une des revendications 1 à 6, **caractérisé en ce qu'**il s'agit d'une ancre de suture (1) et **en ce que** ladite première fente (3a) est destinée à recevoir un ou plusieurs fils de suture (5) pour suturer un tissu tel qu'un ligament ou un tendon.

8. Dispositif d'ancrage osseux (10) selon l'une des revendications 1 à 7, **caractérisé en ce qu'**il comprend une première partie d'ancrage (11) comprenant lesdits premier (12a) et second (12b) bras et lesdites première (14a) et seconde (14b) branches et destinée à être ancrée dans un premier os, et une seconde partie d'ancrage (15), rendue solidaire de la première, par exemple par l'intermédiaire d'une portion rigide centrale (18), et destinée à être ancrée dans un second os, ladite seconde partie d'ancrage (15) pouvant être de forme identique ou différente de ladite première partie d'ancrage (11).

9. Dispositif d'ancrage osseux selon la revendication 8, **caractérisé en ce que** ladite première partie d'ancrage (11) s'étend dans un premier plan et **en ce que** ladite seconde partie d'ancrage (15) s'étend majoritairement dans un second plan formant un angle α, typiquement compris entre 0° et 40°, de préférence entre 0° et 25°, avec ledit premier plan, ladite portion rigide centrale (18) déterminant l'angle α entre lesdits premier et second plans.

10. Dispositif d'ancrage osseux selon la revendication 9, **caractérisé en ce que** ladite seconde partie d'ancrage (15) comprend trois bras (16a, 16b, 17) agencés de sorte que, en position de repos, deux (16a, 16b) desdits bras appelés bras principaux définissent l'envergure de ladite seconde partie d'ancrage perpendiculairement à un axe (y-y') formant ledit angle α avec l'axe (x-x') et s'étendent dans ledit second plan incluant cet axe (y-y') et le troisième (17) desdits bras appelé bras additionnel s'étend dans un troisième plan formant un angle β avec ledit second plan.

11. Dispositif d'ancrage osseux selon la revendication 10, **caractérisé en ce que** lesdits bras principaux (16a, 16b) de ladite seconde partie d'ancrage (15) sont pourvus de crans et **en ce que** ledit bras additionnel (17) de ladite seconde partie d'ancrage (15) est de préférence également pourvu de crans, lesdits crans étant destinés à être en contact avec ledit second os.

12. Dispositif d'ancrage osseux (1) selon l'une des revendications 1 à 11, **caractérisé en ce que** ledit os ou lesdits premier et second os sont choisis parmi une phalange du pied ou de la main.

13. Kit comprenant un dispositif d'ancrage osseux selon l'une des revendications précédentes et un instrument apte à porter ledit dispositif et à le maintenir au moins dans une position dans laquelle l'envergure perpendiculairement à l'axe x-x' de l'ensemble comprenant lesdits premier et second bras et lesdites première et seconde branches est réduite d'au moins 30% par rapport à ladite envergure en position de repos.

## Patentansprüche

1. Knochenverankerungsvorrichtung (1; 10), die einen ersten (2a; 12a) und einen zweiten (2b; 12b) Arm umfasst, die zwischen einander einen ersten Schlitz (3a; 13a) mit einer Tiefe p definieren, der sich entlang einer Achse x-x' erstreckt, wobei der erste (2a; 12a) und der zweite (2b; 12b) Arm einen ersten (4a; 14a) beziehungsweise einen zweiten (4b; 14b) Schenkel tragen, die sich außerhalb des ersten Schlitzes (3a; 13a) erstrecken, wobei der erste Schenkel (4a; 14a) derart angeordnet ist, dass er mit dem Arm (2a, 12a), der ihn trägt, einen zweiten Schlitz (3b; 13b) definiert, und der zweite Schenkel (4b; 14b) derart angeordnet ist, dass er mit dem Arm (2b; 12b), der in trägt, einen dritten Schlitz (3c; 13c) definiert, **dadurch gekennzeichnet, dass** die Vorrichtung (1; 10) derart angeordnet ist, dass in der Ruheposition:
- die Orthogonalprojektion auf der Achse (x-x') des Bodens von jedem von dem zweiten (3b; 13b) und dritten (3c; 13c) Schlitz sich in dem ersten Schlitz (3a; 13a) in einem Abstand vom Boden dieses Schlitzes befindet, der mindestens 10 %, vorzugsweise mindestens 20 %, vorzugsweise mindestens 30 %, vorzugsweise mindestens 40 %, ferner vorzugsweise mindestens 50 %, der Tiefe p beträgt; und
- der Abstand d1, der den Boden des zweiten Schlitzes (3b; 13b) von dem Teil des Endes des ihn definierenden Schenkels (4a; 14a), der am weitesten davon entfernt ist, trennt, und der Abstand d2, der den Boden des dritten Schlitzes (3c; 13c) von dem Teil des Endes des ihn definierenden Schenkels (4b; 14b), der am weitesten davon entfernt ist, trennt, derart sind, dass jedes der Verhältnisse d1/p und d2/p größer oder gleich 0,3, vorzugsweise größer oder gleich 0,4, ferner vorzugsweise größer oder gleich 0,5, ist;
die Vorrichtung (1; 10) dazu geeignet ist, die Spannweite der Anordnung, die den ersten (2a; 12a) und den zweiten (2b; 12b) Arm und den ersten (4a; 14a) und den zweiten (4b; 14b) Schenkel umfasst, senkrecht zur Achse (x-x') ausgehend von der Ruheposition, einerseits durch die elastische Annäherung von jedem der Schenkel (4a, 4b; 14a, 14b) des Arms (2a, 2b; 12a, 12b), der ihn trägt, und andererseits durch die elastische Annäherung des ersten (2a; 12a) und des zweiten Arms (2b; 12b) aneinander für ihre zumindest partielle Einsetzung in einen Knochen zu vermindern.

2. Knochenverankerungsvorrichtung (1; 10) nach Anspruch 1, **dadurch gekennzeichnet, dass** die Spannweite der Anordnung, die den ersten (2a; 12a) und den zweiten (2b; 12b) Arm und den ersten (4a; 14a) und den zweiten (4b; 14b) Schenkel umfasst, senkrecht zur Achse x-x', bei den elastischen Annäherungen um mindestens 30 % in Bezug auf die Spannweite in der Ruheposition verringert werden kann.

3. Knochenverankerungsvorrichtung (1; 10) nach einem der vorhergehenden Ansprüche, **dadurch gekennzeichnet, dass** sie derart gestaltet ist, dass die Anordnung, die ihre Arme (2a, 2b; 12a, 12b) und Schenkel (4a, 4b; 14a, 14b) umfasst, dazu geeignet ist, sich in den Materialien, deren Bruchdruck bei Kompression kleiner oder gleich 10 MPa, vorzugsweise kleiner oder gleich 15 MPa, ist, von einer ersten eingeklappten Position, in der ihre Spannweite senkrecht zur Achse x-x' kleiner oder gleich dem 0,7-, vorzugsweise kleiner oder gleich dem 0,6-, ferner vorzugsweise kleiner oder gleich dem 0,55-Fachen ihrer Spannweite senkrecht zur Achse x-x' in der Ruheposition ist, bis zu einer zweiten Position, in der ihre Spannweite senkrecht zur Achse x-x' größer oder gleich dem 0,75-, vorzugsweise dem 0,80-, vorzugsweise dem 0,85-, vorzugsweise dem 0,90-, vorzugsweise dem 0,95-Fachen ihrer Spannweite senkrecht zur Achse x-x' in der Ruheposition ist, ferner vorzugsweise bis zu ihrer Ruheposition, zu entfalten und dabei nicht in der Lage ist, sich auch nur teilweise in den Materialien zu entfalten, deren Bruchdruck bei Kompression größer oder gleich 200 MPa, vorzugsweise größer oder gleich 100 MPa, vorzugsweise größer oder gleich 50 MPa, ist.

4. Knochenverankerungsvorrichtung (1; 10) nach einem der vorhergehenden Ansprüche, **dadurch gekennzeichnet, dass** sie aus einem superelastischen Material, wie beispielsweise einer Nickel/Titanlegierung, hergestellt ist.

5. Knochenverankerungsvorrichtung (1; 10) nach einem der vorhergehenden Ansprüche, **dadurch gekennzeichnet, dass** in der Ruheposition die Orthogonalprojektion auf der Achse x-x' jedes von dem ersten (4a; 14a) und dem zweiten (4b; 14b) Schenkel sich vollständig oder nahezu vollständig in dem ersten Schlitz (3a; 13a) befindet.

6. Knochenverankerungsvorrichtung (1; 10) nach einem der vorhergehenden Ansprüche, **dadurch gekennzeichnet, dass** sie aus einem Stück besteht.

7. Knochenverankerungsvorrichtung nach einem der Ansprüche 1 bis 6, **dadurch gekennzeichnet, dass** es sich um einen Nahtanker (1) handelt, und dadurch, dass der erste Schlitz (3a) dazu bestimmt ist, einen oder mehrere Nahtfäden (5) aufzunehmen, um ein Gewebe, wie beispielsweise ein Band oder eine Sehne, zu nähen.

8. Knochenverankerungsvorrichtung (10) nach einem der Ansprüche 1 bis 7, **dadurch gekennzeichnet, dass** sie einen ersten Verankerungsteil (11), der den ersten (12a) und den zweiten Arm (12b) und den ersten (14a) und den zweiten Schenkel (14b) umfasst und dazu bestimmt ist, in einem ersten Knochen verankert zu werden, und einen zweiten Verankerungsteil (15) umfasst, der mit dem ersten fest verbunden wird, zum Beispiel mittels eines mittleren starren Abschnitts (18), und dazu bestimmt ist, in einem zweiten Knochen verankert zu werden, wobei der zweite Verankerungsteil (15) eine Form aufweisen kann, die identisch mit derjenigen des ersten Verankerungsteils (11) ist oder sich davon unterscheidet.

9. Knochenverankerungsvorrichtung nach Anspruch 8, **dadurch gekennzeichnet, dass** der erste Verankerungsteil (11) sich in einer ersten Ebene erstreckt, und dadurch, dass der zweite Verankerungsteil (15) sich hauptsächlich in einer zweiten Ebene erstreckt, die einen Winkel α, der typischerweise zwischen 0° und 40°, vorzugsweise zwischen 0° und 25°, beträgt, mit der ersten Ebene bildet, wobei der mittlere starre Abschnitt (18) den Winkel α zwischen der ersten und der zweiten Ebene bestimmt.

10. Knochenverankerungsvorrichtung nach Anspruch 9, **dadurch gekennzeichnet, dass** der zweite Verankerungsteil (15) drei Arme (16a, 16b, 17) umfasst, die derart angeordnet sind, dass in der Ruheposition zwei (16a, 16b) der Arme, die Hauptarme genannt werden, die Spannweite des zweiten Verankerungsteils senkrecht zu einer Achse (y-y') definieren, die den Winkel α mit der Achse (x-x') bildet, und sich in der zweiten Ebene erstrecken, die diese Achse (y-y') umfasst, und der dritte (17) der Arme, der zusätzlicher Arm genannt wird, sich in einer dritten Ebene erstreckt, die einen Winkel β mit der zweiten Ebene bildet.

11. Knochenverankerungsvorrichtung nach Anspruch 10, **dadurch gekennzeichnet, dass** die Hauptarme (16a, 16b) des zweiten Verankerungsteils (15) mit Einkerbungen versehen sind, und dadurch, dass der zusätzliche Arm (17) des zweiten Verankerungsteils (15) vorzugsweise auch mit Einkerbungen versehen ist, wobei die Einkerbungen dazu bestimmt sind, mit dem zweiten Knochen in Kontakt zu sein.

12. Knochenverankerungsvorrichtung (1) nach einem der Ansprüche 1 bis 11, **dadurch gekennzeichnet, dass** der Knochen oder der erste und der zweite Knochen ausgewählt sind aus einem Zehen- oder Fingerknochen.

13. Kit, das eine Knochenverankerungsvorrichtung nach einem der vorhergehenden Ansprüche und ein Instrument umfasst, das dazu geeignet ist, die Vorrichtung zu tragen und sie mindestens in einer Position zu halten, in der die Spannweite senkrecht zur Achse x-x' der Anordnung, die den ersten und den zweiten Arm und den ersten und den zweiten Schenkel umfasst, in Bezug auf die Spannweite in der Ruheposition um mindestens 30 % vermindert ist.

## Claims

1. Bone anchoring device (1; 10) comprising a first (2a; 12a) and a second (2b; 12b) arm defining therebetween a first slot (3a; 13a) of depth p extending along an axis x-x', said first (2a; 12a) and second (2b; 12b) arms respectively bearing a first (4a; 14a) and a second (4b; 14b) branch extending outside of said first slot (3a; 13a), said first branch (4a; 14a) being arranged so as to define with the arm (2a, 12a) which bears it a second slot (3b; 13b) and said second branch (4b; 14b) being arranged so as to define with the arm (2b; 12b) which bears it a third slot (3c; 13c), **characterized in that** said device (1; 10) is arranged such that, in the rest position:
- the orthogonal projection on the axis (x-x') of the bottom of each of the second (3b; 13b) and third (3c; 13c) slots is located in said first slot (3a; 13a) at a distance from the bottom of this slot of at least 10%, preferably at least 20%, preferably at least 30%, preferably at least 40%, even more preferably at least 50%, of the depth p; and
- the distances d1 separating the bottom of said second slot (3b, 13b) from the part of the end of the branch (4a; 14a) which defines it which is the furthest away from it, and d2 separating the bottom of said third slot (3c; 13c) from the part of the end of the branch (4b; 14b) which defines it which is the furthest away from it, are such that each of the ratios d1/p and d2/p is greater than or equal to 0.3, preferably greater than or equal to 0.4, even more preferably greater than or equal to 0.5;
said device (1; 10) being able to reduce the span of the assembly comprising said first (2a; 12a) and second (2b; 12b) arms and said first (4a; 14a) and second (4b; 14b) branches perpendicular to the axis (x-x') from said rest position, on the one hand by elastically bringing each of the branches (4a, 4b; 14a, 14b) closer to the arm (2a, 2b; 12a, 12b) which bears it, and on the other hand by elastically bringing said first (2a; 12a) and second (2b; 12b) arms closer together for the insertion thereof, at least in part, into a bone.

2. Bone anchoring device (1; 10) as claimed in claim 1, **characterised in that** the span of the assembly comprising said first (2a; 12a) and second (2b; 12b) arms and said first (4a; 14a) and second (4b; 14b) branches perpendicular to the axis x-x' can, when said elements are elastically brought closer together, be reduced by at least 30% with respect to said span in the rest position.

3. Bone anchoring device (1; 10) as claimed in any one of the preceding claims, **characterised in that** it is designed such that the assembly comprising its arms (2a, 2b; 12a, 12b) and branches (4a, 4b; 14a, 14b) is able to expand from a first folded position in which its span perpendicular to the axis x-x' is less than or equal to 0.7, preferably less than or equal to 0.6, even more preferably less than or equal to 0.55, times its span perpendicular to the axis x-x' in the rest position, to a second position in which its span perpendicular to the axis x-x' is greater than or equal to 0.75, preferably 0.80, preferably 0.85, preferably 0.90, preferably 0.95, times its span perpendicular to the axis x-x' in the rest position, even more preferably to its rest position, into the materials in which the compression failure pressure is less than or equal to 10 MPa, preferably less than or equal to 15 MPa, whilst being incapable of expanding even partially into materials in which the compression failure pressure is greater than or equal to 200 MPa, preferably greater than or equal to 100 MPa, preferably greater than or equal to 50 MPa.

4. Bone anchoring device (1; 10) as claimed in any one of the preceding claims, **characterised in that** it is produced from a superelastic material such as a nickel/titanium alloy.

5. Bone anchoring device (1; 10) as claimed in any one of the preceding claims, **characterised in that** in said rest position the orthogonal projection on the axis x-x' of each of said first (4a; 14a) and second (4b; 14b) branches is located fully or almost fully in said first slot (3a; 13a).

6. Bone anchoring device (1; 10) as claimed in any one of the preceding claims, **characterised in that** it is in one piece.

7. Bone anchoring device as claimed in any one of claims 1 to 6, **characterised in that** it is a suture anchor (1) and **in that** said first slot (3a) is intended to receive one or more suture threads (5) to suture a tissue such as a ligament or tendon.

8. Bone anchoring device (10) as claimed in any one of claims 1 to 7, **characterised in that** it comprises a first anchoring part (11) comprising said first (12a) and second (12b) arms and said first (14a) and second (14b) branches and intended to be anchored in a first bone, and a second anchoring part (15), made fixedly attached to the first part, for example via a central rigid portion (18), and intended to be anchored in a second bone, the shape of said second anchoring part (15) being able to be identical to or different from that of said first anchoring part (11).

9. Bone anchoring device as claimed in claim 8, **characterised in that** said first anchoring part (11) extends in a first plane and **in that** said second anchoring part (15) extends mostly in a second plane forming an angle α, typically between 0° and 40°, preferably between 0° and 25°, with said first plane, said central rigid portion (18) determining the angle α between said first and second planes.

10. Bone anchoring device as claimed in claim 9, **characterised in that** said second anchoring part (15) comprises three arms (16a, 16b, 17) arranged such that, in the rest position, two (16a, 16b) of said arms, called main arms, define the span of said second anchoring part perpendicular to an axis (y-y') forming said angle α with the axis (x-x') and extend in said second plane including this axis (y-y') and the third (17) of said arms, called additional arm, extends in a third plane forming an angle β with said second plane.

11. Bone anchoring device as claimed in claim 10, **characterised in that** said main arms (16a, 16b) of said second anchoring part (15) are provided with notches and **in that** said additional arm (17) of said second anchoring part (15) is preferably likewise provided with notches, said notches being intended to be in contact with said second bone.

12. Bone anchoring device (1) as claimed in any one claims 1 to 11, **characterised in that** said bone or said first and second bones are selected from a phalanx of the foot or of the hand.

13. Kit comprising a bone anchoring device as claimed in any one of the preceding claims and an instrument able to bear said device and keep it at least in a position in which the span perpendicular to the axis x-x' of the assembly comprising said first and second arms and said first and second branches is reduced by at least 30% with respect to said span in the rest position.
